(19) Europäisches Patentamt
European Patent Office
Office européen des brevets

(11) EP 3 838 965 B1

(12) # EUROPÄISCHE PATENTSCHRIFT

(45) Veröffentlichungstag und Bekanntmachung des Hinweises auf die Patenterteilung:
**03.09.2025 Patentblatt 2025/36**

(21) Anmeldenummer: **19217806.9**

(22) Anmeldetag: **19.12.2019**

(51) Internationale Patentklassifikation (IPC):
***C08G 18/64*** *(2006.01)* ***C08G 18/10*** *(2006.01)*
***C08G 18/32*** *(2006.01)* ***A61F 13/01*** *(2024.01)*
***C08G 18/30*** *(2006.01)* ***A61K 31/718*** *(2006.01)*
***A61K 31/785*** *(2006.01)* ***C08K 3/16*** *(2006.01)*
*C08G 101/00 (2006.01)* *C08G 18/75 (2006.01)*
*C08G 18/48 (2006.01)*

(52) Gemeinsame Patentklassifikation (CPC):
(C-Sets verfügbar)
**C08G 18/6484; A61F 13/01012; A61F 13/01017; A61K 31/785; C08G 18/10; C08G 18/302; C08G 18/3206; C08K 3/16;** C08G 18/485; C08G 18/755; C08G 2101/00; C08G 2110/0083; C08G 2210/00 (Forts.)

(54) **VERFAHREN ZUR HERSTELLUNG EINES STÄRKEDERIVATHALTIGEN POLYMERS FÜR MEDIZINISCHE ZWECKE, INSBESONDERE FÜR DIE WUNDBEHANDLUNG**

METHOD FOR THE PRODUCTION OF A STARCH DERIVATIVE-CONTAINING POLYMER FOR MEDICAL PURPOSES, IN PARTICULAR FOR TREATING WOUNDS

PROCÉDÉ DE FABRICATION D'UN POLYMÈRE CONTENANT DE L'AMIDON À DES FINS MÉDICALES, EN PARTICULIER POUR LE TRAITEMENT DES PLAIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.06.2021 Patentblatt 2021/25**

(73) Patentinhaber: **Paul Hartmann AG**
**89522 Heidenheim (DE)**

(72) Erfinder: **KETTEL, Markus**
**89520 Heidenheim (DE)**

(74) Vertreter: **Paul Hartmann AG**
**Patents & Licensing**
**Paul-Hartmann-Straße 12**
**89522 Heidenheim (DE)**

(56) Entgegenhaltungen:
**EP-A1- 3 235 520**
**CN-A- 110 577 627**
**JP-A- 2010 254 985**
**KR-B1- 101 879 643**
**US-A- 4 773 409**
**US-A- 5 065 752**
**US-B2- 7 538 257**

EP 3 838 965 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C08G 18/10, C08G 18/302;**
**C08G 18/10, C08G 18/3206;**
**C08G 18/10, C08G 18/64;**
**C08K 3/16, C08L 75/08**

## Beschreibung

Die vorliegende Erfindung betrifft ein Verfahren zur Herstellung eines Polymers für medizinische Zwecke, insbesondere für die Wundbehandlung. Die Erfindung betrifft auch das mit dem Verfahren erhaltene Polymer als solches sowie dessen Einsatz in der Medizin und in Wundauflagen.

Ein Hydrogel ist ein disperses System aus mindestens einer festen hydrophilen Phase und einer flüssigen, wässrigen Phase. Wundverbände mit einer Hydrogelschicht sind im Stand der Technik bekannt. Derartige Wundverbände können sowohl Wundexsudat absorbieren als auch die Wunde befeuchten und so die Wundheilung beschleunigen. Die feste Phase der Hydrogele wird dabei häufig aus vollsynthetischen Stoffen hergestellt, welche im Hinblick auf die Biokompatibilität, die Nachhaltigkeit sowie die Umweltverträglichkeit problematisch sein können.

CN 110 577 627 A und KR 101 879 643 B1 offenbaren Verfahren zur Herstellung von Polyurethanschäumen unter Einsatz von unter anderem Isocyanat-terminierten Präpolymeren, Wasser und Carboxymethylcellulose.

EP 3 235 520 A1 offenbart ein Verfahren zur Herstellung eines Partikel umfassenden Schaums. In einem ersten Schritt des Verfahrens wird eine Mischung umfassend ein NCOterminiertes Polyurethan-Präpolymer und Wasser absorbierende Partikel hergestellt. In einem zweiten Schritt des Verfahrens wird diese Mischung mit Wasser zusammengeführt. Bei den Wasser absorbierenden Partikeln kann es sich um einen vernetzten Stärkeether handeln.

JP 2010 254985 A offenbart ein Herstellungsverfahren für ein wasserlösliches Urethanmodifiziertes Cellulosederivat. Dabei wird ein Cellulosederivat mit einem Isocyanat-terminierten Präpolymer zur Reaktion gebracht.

US 7,538,257 B2 offenbart eine Zusammensetzung für die Wundbehandlung, die eine teilweise gehärtete Polyurethanflüssigkeit enthält. Die Zusammensetzung kann direkt in eine Wunde injiziert werden, wo sie aushärtet und einen wundfüllenden Verband bildet.

US 5,065,752 A offenbart einen hydrophilen Schaumstoff, umfassend das In-situ-Reaktionsprodukt eines isocyanatverkappten Polyether-Präpolymers mit einem hydrophilen, Wasser-absorbierenden Mittel, einem Hilfsstoff umfassend einen Alkohol, einem Feuchthaltemittel und Wasser.

US 4,773,409 A offenbart einen okklusiven Wundverband umfassend einen flexiblen, geschlossenzelligen Polyurethanschaum, der etwa 5 bis etwa 50 Gewichtsprozent des Schaums an einem oder mehreren wasserdispergierbaren, wasserquellbaren und/oder wasserabsorbierenden Mitteln enthält.

Aufgabe der vorliegenden Erfindung war es, ein neues Material für medizinische Zwecke bereitzustellen. Insbesondere war es die Aufgabe der vorliegenden Erfindung, ein neues Material für die Wundbehandlung zur Verfügung zu stellen. Das Material sollte dabei insbesondere biokompatibler, nachhaltiger und umweltverträglicher sein. Es hat sich gezeigt, dass ein Polymer, hergestellt nach dem Verfahren von Anspruch 1, die zuvor genannten Aufgaben und Anforderungen erfüllen kann.

Das erfindungsgemäße Polymer wird mit den nachfolgenden Schritten hergestellt:

i. Bereitstellen eines Isocyanat-terminierten Präpolymers enthaltend Polyalkylenoxideinheiten.
ii. Lösen eines Stärkederivats in einer wässrigen Flüssigkeit, um eine wässrige, stärkederivathaltige Zubereitung zu erhalten.
iii. Mischen des Präpolymers, der wässrigen, stärkederivathaltigen Zubereitung und optional einer oder mehrerer weiterer Komponenten, um eine Reaktionsmischung zu erhalten.
iv. Umsetzen des Präpolymers in der Reaktionsmischung, um das Polymer zu erhalten.

Der Anteil an dem Präpolymer bezogen auf das Gesamtgewicht der Reaktionsmischung beträgt erfindungsgemäß 5 bis 80 Gew.-%, insbesondere 20 bis 80 Gew.-%.

Der Anteil an dem Stärkederivat bezogen auf das Gesamtgewicht der Reaktionsmischung beträgt erfindungsgemäß 0,5 bis 5 Gew.-%, insbesondere 0,5 bis 2 Gew.-%.

Der Anteil der wässrigen Flüssigkeit bezogen auf das Gesamtgewicht der Reaktionsmischung beträgt erfindungsgemäß 20 bis 90 Gew.-%, insbesondere 20 bis 75 Gew.-%.

Der Anteil der einen oder mehreren weiteren Komponenten bezogen auf das Gesamtgewicht der Reaktionsmischung beträgt erfindungsgemäß 0 bis 30 Gew.-%.

Erfindungsgemäß liegt das Polymer zudem als ein Gel, ein Gelschaum oder ein Schaumstoff vor.

Die Reihenfolge der Schritte i. und ii. kann auch vertauscht werden und ist nicht erfindungswesentlich.

Das Isocyanat-terminierte Präpolymer wird im vorliegenden Dokument auch verkürzt als Präpolymer bezeichnet. Wird also im vorliegenden Dokument auf das Präpolymer Bezug genommen, so ist insofern nicht anders angegeben das Isocyanat-terminierte Präpolymer gemeint.

Stärkederivate im Sinne der vorliegenden Erfindung sind chemisch oder physikalisch modifizierte Stärken. Insbesondere werden die Stärkederivate mittels polymeranaloger Reaktionen erhalten, wobei ein Teil der Hydroxyl-Gruppen der Glucose-Einheiten der Stärke durch Veretherung oder Veresterung umgewandelt sein können.

Kennzeichnend für die vorliegende Erfindung ist, dass das Stärkederivat in das Polymer eingeschlossen ist. Dies

wird insbesondere dadurch erreicht, indem das Präpolymer in der Reaktionsmischung mit dem Stärkederivat reagieren kann und somit in dem Polymer zumindest ein Teil des Stärkederivats oder vorzugsweise die Gesamtmenge des Stärkederivats kovalent gebunden vorliegt. Damit das Präpolymer mit dem Stärkederivat reagieren kann, weist das Stärkederivat üblicherweise entsprechende funktionelle Gruppen wie zum Beispiel Hydroxyl-Gruppen auf. Das Stärkederivat ist also üblicherweise so ausgewählt, dass es Hydroxyl-Gruppen oder andere mit dem Präpolymer reaktionsfähige funktionelle Gruppen enthält. Die bei der Reaktion des Präpolymers mit einem solchen Stärkederivat ablaufenden Reaktionsmechanismen werden nachfolgend noch genauer beschrieben.

Grundsätzlich wird mit dem erfindungsgemäßen Polymer ein hydratisiertes Polyurethan-Hydrogel-System beziehungsweise ein hydratisiertes Polyurethan-Schaum-System bereitgestellt, in dessen quervernetzte Polymerstruktur ein Stärkederivat insbesondere durch kovalenten Einbau fest eingeschlossen ist. Stärkederivate sind vergleichsweise kostengünstig und können auf Basis von nachwachsenden, natürlich vorkommenden Rohstoffen, zum Beispiel Kartoffeln, Mais, Reis und Weizen, gewonnen werden. Stärkederivate können viele vorteilhafte Eigenschaften besitzen. So können Stärkederivate zum Beispiel ungiftig, biokompatibel und bioabbaubar sein. Indem für die Herstellung des erfindungsgemäßen Polymers ein Stärkederivat verwendet wird und dadurch auf weniger vollsynthetische Ausgangstoffe zurückgegriffen werden muss, kann die Biokompatibilität, Nachhaltigkeit und Umweltverträglichkeit im Vergleich zu einem konventionellen Hydrogel verbessert werden. Das erfindungsgemäße Polymer kann daher eine bessere Verträglichkeit für menschliche und tierische Gewebe aufweisen und die Umwelt schonen. Das erfindungsgemäße Polymer kann mindestens ebenso gut wie ein konventionelles Hydrogel ein die Wundheilung unterstützendes feuchtes Wundklima erzeugen, indem es Wundexsudat absorbieren und/oder Feuchtigkeit an die Wunde abgeben kann. Es verklebt dabei nicht mit der Wunde. Diese Eigenschaften ermöglichen eine effektive und schonende Behandlung von vielen unterschiedlichen Wundtypen. Ein weiterer Vorteil des erfindungsgemäßen Polymers ist seine einfache Herstellbarkeit.

In einer besonders bevorzugten Ausführungsform der Erfindung ist das Präpolymer mindestens dreiarmig (insbesondere genau dreiarmig) verzweigt und die Polyalkylenoxideinheiten werden durch Polyethylenoxid- und/oder Polypropylenoxideinheiten gebildet. Im obigen Schritt i. wird dann also ein mindestens dreiarmig verzweigtes Isocyanat-terminiertes Präpolymer enthaltend Polyethylenoxid- und/oder Polypropylenoxideinheiten bereitgestellt. Dabei kann das Gewichtsverhältnis von Ethylenoxid- zu Propylenoxideinheiten 3 : 1 bis 7 : 1 betragen. Mit einer solchen Verbindung lässt sich das Polymer besonders gut herstellen.

Ein Isocyanat-terminiertes Präpolymer gemäß der zuvor genannten besonders bevorzugten Ausführungsform ist beispielsweise ein dreiarmiges Copolymer aus Ethylenoxid- und Propylenoxideinheiten, welches endständig jeweils mit einem Molekül Isophorondiisocyanat umgesetzt wurde. Es weist einen Gehalt reaktiver Isocyanatendgruppen (NCO-Gruppen) von 2,5 % bis 4,0 %, bevorzugt 3,0 % bis 3,4 %, besonders bevorzugt 3,2 %, und ein molares Verhältnis von Ethylenoxideinheiten zu Propylenoxideinheiten von 3 : 1 bis 4 : 1 auf. Ein derartiges Isocyanat-terminiertes Präpolymer mit aliphatischen Isocyanat-Gruppen ist zum Beispiel als Aquapol® PI-13000-31 (Carpenter; Richmond, USA) kommerziell erhältlich.

Die nachfolgende Abbildung veranschaulicht die schematische Struktur eines dreiarmig verzweigten Isocyanat-terminierten Präpolymers enthaltend Polyethylenoxid- und Polypropylenoxideinheiten (wie bei Aquapol®). Ein Glycerol-Molekül bildet das Zentrum des Präpolymers. Die drei "Arme" des Präpolymers mit jeweils einer endständigen Isocyanat-Gruppe sind mit den Hydroxyl-Gruppen des Glycerol-Moleküles verknüpft. Das Glycerol-Molekül selber ist in der Abbildung nicht dargestellt. Es würde sich in der rechten Bildhälfte dort befinden, wo die als Wellenlinie schematisiert dargestellten drei "Arme" aufeinandertreffen. In der linken Bildhälfte ist die chemische Struktur eines "Arms" genauer dargestellt.

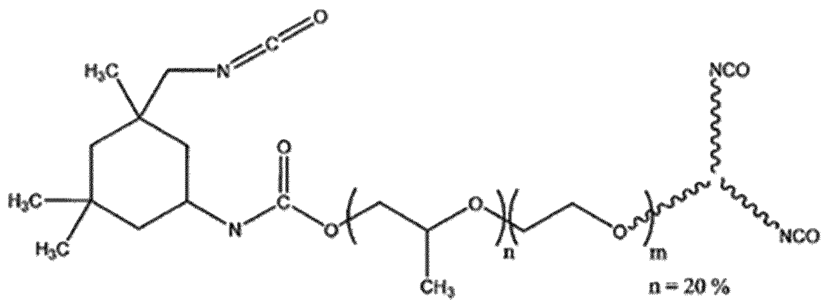

n = 20 %
m = 80 %

Das verwendete Stärkederivat kann Natriumstärkeglycolat sein (engl. systematischer Name: starch, carboxymethyl ether, sodium salt; CAS Nummer 9063-38-1). Dieses Stärkederivat kann wasserlöslich sein sowie Hydroxyl-Gruppen enthalten und daher für das erfindungsgemäße Herstellungsverfahren geeignet sein. Ein für die vorliegende Erfindung geeignetes Natriumstärkeglycolat wird zum Beispiel unter der Bezeichnung "Explotab PCF" von der Firma JRS Pharma (Rosenberg, Deutschland) im Handel angeboten.

Die wässrige Flüssigkeit hat einen Wassergehalt von mindestens 50 Gew.-%. Normalerweise hat die vorliegend eingesetzte wässrige Flüssigkeit jedoch einen deutlich höheren Wassergehalt, zum Beispiel mindestens 80 Gew.-%, mindestens 85 Gew.-%, mindestens 90 Gew.-% oder mindestens 95 Gew.-%. Bevorzugt handelt es sich bei der wässrigen Flüssigkeit um Wasser, dem gegebenenfalls zum besseren Lösen des Stärkederivats noch eine Base zugesetzt ist. Die Base kann insbesondere Natriumhydroxid sein. Insbesondere kann die wässrige Flüssigkeit jedoch aus Wasser bestehen. Die wässrige Flüssigkeit wird anspruchsgemäß dazu eingesetzt, um das Stärkederivat in Lösung zu bringen. Dabei wird eine wässrige, stärkederivathaltige Zubereitung erhalten, welche gleichfalls eine Flüssigkeit sein kann. Die Viskosität der Zubereitung kann von der Konzentration des Stärkederivats abhängen.

Bei den weiteren Komponenten, die mit dem Präpolymer und der wässrigen, stärkederivathaltigen Zubereitung im Herstellungsschritt iii. optional gemischt werden können, kann es sich zum Beispiel um ein Feuchthaltemittel oder ein anorganisches Salz handeln. Damit können die chemischen und physikalischen Eigenschaften des Polymers beeinflusst und für die jeweilige Anwendung angepasst werden. Dennoch ist die Anwesenheit der weiteren Komponenten nicht zwingend erforderlich, weshalb sie vorliegend auch als optional ausgewiesen sind. So kann es vorgesehen sein, dass zum Erhalt der Reaktionsmischung nur das Präpolymer mit der wässrigen, stärkederivathaltigen Zubereitung gemischt wird. Die Reaktionsmischung besteht dann aus dem Präpolymer und der wässrigen, stärkederivathaltigen Zubereitung.

Als Feuchthaltemittel kann Ethylenglykol, Propylenglykol, PEG300, PEG2000, Glycerol, Saccharose oder Sorbitol verwendet werden. Besonders bevorzugt ist dabei Glycerol. Die Feuchthaltemittel können die Absorptionsfähigkeit des Polymers erhöhen sowie den Feuchtigkeitsverlust des Polymers verringern.

Als anorganisches Salz kann Natriumchlorid, Kaliumchlorid, Magnesiumchlorid, Calciumchlorid oder eine Mischung aus mindestens zwei dieser Salze verwendet werden. Besonders bevorzugt ist dabei Natriumchlorid als alleiniges Salz. Eine bevorzugte Salzmischung enthält Natriumchlorid, Kaliumchlorid und Calciumchlorid. Diese drei Salze werden auch zur Herstellung der Ringerlösung verwendet. Die anorganischen Salze können den Elektrolyt-Gehalt in einem von einer Wunde abgegebenen Wundserum simulieren. Damit stellt das Polymer einer Wunde ein besonders wundheilungsförderndes Klima zur Verfügung.

Die weitere Komponente kann auch ein Treibmittel sein. Vorzugsweise umfasst das Treibmittel Natriumhydrogencarbonat. Insbesondere besteht das Treibmittel aus Natriumhydrogencarbonat und einer Säure wie zum Beispiel Citronensäure, Weinsäure oder Salzsäure. Mit dem Treibmittel kann die Porosität und damit die Saugkraft des Polymers erhöht werden. Der Einsatz des Treibmittels ist insbesondere dann sinnvoll und vorteilhaft, wenn ein Schaumstoff hergestellt werden soll.

Die nachfolgenden Bereiche sind erfindungsgemäß für die Herstellung des Polymers mit einem kovalent gebundenen Stärkederivat vorgesehen:
Der Anteil an dem Präpolymer bezogen auf das Gesamtgewicht der Reaktionsmischung beträgt 5 bis 80 Gew.-%. Insbesondere beträgt der Anteil an dem Präpolymer bezogen auf das Gesamtgewicht der Reaktionsmischung 20 bis 80 Gew.-%.

Der Anteil an dem Stärkederivat bezogen auf das Gesamtgewicht der Reaktionsmischung beträgt 0,5 bis 5 Gew.-%. Insbesondere beträgt der Anteil an dem Stärkederivat bezogen auf das Gesamtgewicht der Reaktionsmischung 0,5 bis 2 Gew.-%.

Der Anteil der wässrigen Flüssigkeit bezogen auf das Gesamtgewicht der Reaktionsmischung beträgt 20 bis 90 Gew.-%. Insbesondere beträgt der Anteil der wässrigen Flüssigkeit bezogen auf das Gesamtgewicht der Reaktionsmischung 20 bis 75 Gew.-%.

Der Anteil der einen oder mehreren weiteren Komponenten bezogen auf das Gesamtgewicht der Reaktionsmischung beträgt 0 bis 30 Gew.-%. Der Bereich von 0 bis 30 Gew.-% bezieht sich dabei auf die Summe aller vorhandenen weiteren Komponenten.

Dabei ergeben der Anteil an dem Präpolymer, der Anteil an dem Stärkederivat, der Anteil der wässrigen Flüssigkeit und der Anteil der weiteren Komponenten zusammen 100 Gew.-%. Wenn keine weiteren Komponenten vorgesehen sind (die optionalen weiteren Komponenten also fehlen), ergeben der Anteil an dem Präpolymer, der Anteil an dem Stärkederivat und der Anteil der wässrigen Flüssigkeit zusammen 100 Gew.-%.

Chemisch gesehen handelt es sich bei den hier vorgeschlagenen Polymeren üblicherweise um Hydrogele. Ein Hydrogel ist wie bereits eingangs erwähnt ein disperses System aus mindestens einer festen hydrophilen Phase und einer flüssigen Phase, nämlich Wasser. Dabei bildet die feste Phase ein schwammartiges, dreidimensionales polymeres Netzwerk, welches durch das Wasser ausgefüllt ist. In Abhängigkeit von dem Mengenverhältnis Präpolymer zu wässriger Flüssigkeit in der Reaktionsmischung unterscheidet sich das äußere Erscheinungsbild der Polymere. Erfindungsgemäß liegt das Polymer hinsichtlich seines äußeren Erscheinungsbildes als ein Gel, als ein Gelschaum oder als ein Schaumstoff vor.

Wenn das Polymer als ein Gel vorliegt, enthält es keine oder nur wenige Hohlräume beziehungsweise Zellen. Das Polymer kann dann eine zusammenhängende, diskrete Schicht ausbilden. Das Polymer kann als Gel auch transparent sein. Wenn das Polymer als ein Gel vorliegt, weist es also ein für Hydrogele übliches äußeres Erscheinungsbild auf. Das gelartige Polymer hat ein hohes Wasserabgabevermögen und ist somit besonders gut für die Behandlung

von trockenen Wunden geeignet.

Wenn das Polymer als ein Schaumstoff vorliegt, ist es porös und enthält eine Vielzahl offener und/oder geschlossener Hohlräume beziehungsweise Zellen. Das Schaumstoff-Polymer ist normalerweise weiß und undurchsichtig sowie im Vergleich zu einem Gelpolymer mit gleicher Masse voluminöser. Außerdem enthält das Schaumstoff-Polymer gegenüber einem Gelpolymer mit gleicher Masse einen höheren festen polymeren Anteil und weniger Wasser. Das schaumstoffartige Polymer hat ein hohes Wasseraufnahmevermögen und ist somit besonders gut für die Behandlung von stark exsudierenden Wunden geeignet.

Die als "Gelschaum" bezeichnete Variante des Polymers stellt eine Übergangsform zwischen Gel und Schaumstoff dar. Der "Gelschaum" kann auch als ein vorgequollener hydrophiler Schaumstoff aufgefasst werden. Der Gelschaum kann sowohl vergleichsweise viel Wasser abgeben als auch vergleichsweise viel Wasser aufnehmen und ist somit für die Behandlung von unterschiedlichsten Wundtypen geeignet.

Nachfolgend werden drei alternative Ausführungsformen der Erfindung genannt, in denen die zuvor genannten Bereiche für das Präpolymer, das Stärkederivat und die wässrige Flüssigkeit weiter spezifiziert werden, so dass die Polymere entweder als Gel, Gelschaum oder Schaumstoff vorliegen. Der Übergang von einem Gel zu einem Gelschaum bis hin zu einem Schaumstoff kann insbesondere dadurch erzielt werden, indem der Anteil an Präpolymer erhöht und der Anteil an der wässrigen Flüssigkeit verringert wird.

Wenn nach dem Umsetzen des Präpolymers in der Reaktionsmischung ein Polymer in der Form eines Gels erhalten werden soll, können die Bereiche wie folgt gewählt werden:
Der Anteil an dem Präpolymer bezogen auf das Gesamtgewicht der Reaktionsmischung beträgt 5 bis 35 Gew.-%.

Der Anteil an dem Stärkederivat bezogen auf das Gesamtgewicht der Reaktionsmischung beträgt vorzugsweise 1,7 bis 5 Gew.-% beträgt.

Der Anteil der wässrigen Flüssigkeit bezogen auf das Gesamtgewicht der Reaktionsmischung beträgt 65 bis 90 Gew.-%.

Wenn nach dem Umsetzen des Präpolymers in der Reaktionsmischung ein Polymer in der Form eines Gelschaums erhalten werden soll, können die Bereiche wie folgt gewählt werden:
Der Anteil an dem Präpolymer bezogen auf das Gesamtgewicht der Reaktionsmischung beträgt 36 bis 45 Gew.-%.

Der Anteil an dem Stärkederivat bezogen auf das Gesamtgewicht der Reaktionsmischung beträgt vorzugsweise 1,5 bis 1,7 Gew.-%.

Der Anteil der wässrigen Flüssigkeit bezogen auf das Gesamtgewicht der Reaktionsmischung beträgt 55 bis 60 Gew.-%.

Wenn nach dem Umsetzen des Präpolymers in der Reaktionsmischung ein Polymer in der Form eines Schaumstoffs erhalten werden soll, können die Bereiche wie folgt gewählt werden:
Der Anteil an dem Präpolymer bezogen auf das Gesamtgewicht der Reaktionsmischung beträgt 50 bis 80 Gew.-%.

Der Anteil an dem Stärkederivat bezogen auf das Gesamtgewicht der Reaktionsmischung beträgt vorzugsweise 0,5 bis 1,3 Gew.-%.

Der Anteil der wässrigen Flüssigkeit bezogen auf das Gesamtgewicht der Reaktionsmischung beträgt 22 bis 50 Gew.-%.

Falls ein Feuchthaltemittel wie zum Beispiel Glycerol vorgesehen ist, so kann dieses vorteilhafterweise einen Anteil von 10 bis 25 Gew.-%, insbesondere 15 bis 25 Gew.-%, bezogen auf das Gesamtgewicht der Reaktionsmischung haben.

Falls ein anorganisches Salz wie zum Beispiel Natriumchlorid vorgesehen ist, so kann dieses vorteilhafterweise einen Anteil von 0,1 bis 5 Gew.-%, bevorzugt von 0,1 bis 3 Gew.-% und besonders bevorzugt von 0,5 bis 1,5 Gew.-% bezogen auf das Gesamtgewicht der Reaktionsmischung haben.

Falls ein Treibmittel wie zum Beispiel Natriumhydrogencarbonat vorgesehen ist, so kann dieses vorteilhafterweise einen Anteil von 1 bis 10 Gew.-%, bevorzugt von 2 bis 10 Gew.-% und besonders bevorzugt von 5 bis 10 Gew.-% bezogen auf das Gesamtgewicht der Reaktionsmischung haben.

Bei dem Umsetzen des Präpolymers finden Reaktionen statt, an denen die Isocyanat-Gruppen des Präpolymers beteiligt sind. Das Umsetzen des Präpolymers setzt üblicherweise automatisch ein, sobald die Reaktionsmischung hergestellt worden ist. Das Umsetzen des Präpolymers in der Reaktionsmischung kann als ein Polymerisationsprozess verstanden werden, der zur Ausbildung des Polymers führt.

Normalerweise erfolgt das Umsetzen des Präpolymers in der Reaktionsmischung im Wesentlichen vollständig. Es liegen dann keine für menschliche und tierische Zellen toxischen Isocyanat-Gruppen in dem Polymer mehr vor. Der Nachweis ob das Präpolymer in der Reaktionsmischung im Wesentlichen vollständig umgesetzt worden ist kann beispielsweise mit Hilfe der IR-Spektroskopie erbracht werden. Eine fehlende Absorptionsbande bei 2260 $cm^{-1}$ für die Isocyanat-Gruppe dient dabei als Nachweis für ein vollständiges Umsetzen des Präpolymers.

An dem Umsetzen des Präpolymers in der Reaktionsmischung können insbesondere die folgenden Reaktionen a) bis c) beteiligt sein, wobei Reaktion a) schneller als Reaktion b) abläuft:

a) Reaktion einer Isocyanat-Gruppe des Präpolymers mit einer Hydroxyl-Gruppe des Stärkederivats, wobei eine Urethan-Bindung gebildet wird.

b) Reaktion einer Isocyanat-Gruppe des Präpolymers mit einem Wassermolekül, wobei die Isocyanat-Gruppe unter Freisetzung von Kohlendioxid in eine Amin-Gruppe umgewandelt wird.

c) Reaktion einer Isocyanat-Gruppe des Präpolymers mit einer nach Reaktion b) gebildeten Amin-Gruppe, wobei eine Harnstoff-Bindung gebildet wird.

Welche der Reaktionen a) bis c) bei dem Umsetzen des Präpolymers in der Reaktionsmischung ablaufen beziehungsweise überwiegen, hängt unter anderem von den zuvor genannten Reaktionsgeschwindigkeiten sowie den Mengenverhältnissen von Präpolymer, Stärkederivat und Wasser ab.

Insbesondere ist die obige Reaktionen a) an dem Umsetzen des Präpolymers beteiligt, so dass wie eingangs erwähnt zumindest ein Teil des Stärkederivats oder vorzugsweise die Gesamtmenge des Stärkederivats kovalent an das Präpolymer gebunden wird. Falls nur ein Teil des Stärkederivats kovalent gebunden wird (zum Beispiel wenn wenig Präpolymer und viel Stärkederivat eingesetzt wird), ist der verbleibende, nicht kovalent gebundene Teil des Stärkederivats in der Regel gleichfalls fest in die Struktur des Polymers eingeschlossen und kann aus diesem nicht austreten. Hierfür können hydrophile Wechselwirkungen der Stärkederivat-Moleküle mit dem Polymer verantwortlich sein. Nicht kovalent gebundenes Stärkederivat kann aber auch einfach deshalb in das Polymer eingeschlossen werden, weil die Stärkederivat-Moleküle größer als die "Maschen" des Polymernetzes sind.

Der Nachweis ob das Präpolymer mit dem Stärkederivat gemäß Reaktion a) umgesetzt worden ist kann beispielsweise mit Hilfe der IR-Spektroskopie erbracht werden. Hierfür kann die Absorption bei 1654 $cm^{-1}$ (für die Harnstoff-Bindung) beziehungsweise 1714 $cm^{-1}$ (für die Urethan-Bindung) im IR-Spektrum des Polymers ausgewertet werden. Insbesondere kann der kovalente Einbau des Stärkederivats an einer stärkeren Absorptionsbande bei 1714 $cm^{-1}$ (Urethan-Bindung) im Vergleich zu einem Polymer mit gleichem Präpolymergehalt, aber ohne Stärkederivat erkannt werden.

An dem Umsetzen des Präpolymers in der Reaktionsmischung können also eine oder mehrere Reaktionen ausgewählt aus der Gruppe umfassend Reaktion a), Reaktion b) und Reaktion c) beteiligt sein, wobei zumindest Reaktion a) an dem Umsetzen des Präpolymers beteiligt ist. Die zuvor genannte Gruppe kann hierbei auch noch weitere, an der Umsetzung des Präpolymers beteiligte Reaktionen umfassen.

Es kann vorgesehen sein, dass nur die zuvor genannten Reaktionen a) bis c) an dem Umsetzen des Präpolymers beteiligt sind. An dem Umsetzen des Präpolymers in der Reaktionsmischung wären dann eine oder mehrere Reaktionen ausgewählt aus der Gruppe bestehend aus Reaktion a), Reaktion b) und Reaktion c) beteiligt, wobei zumindest Reaktion a) an dem Umsetzen des Präpolymers beteiligt ist. Das Umsetzen des Präpolymers kann auf die Reaktionen a) bis c) beschränkt sein, wenn die wässrige Flüssigkeit aus Wasser besteht und die Reaktionsmischung aus dem Präpolymer und der wässrigen, stärkederivathaltigen Zubereitung besteht.

In der Regel ist es weiterhin vorgesehen, dass in der Reaktionsmischung kein Amin-terminiertes Präpolymer, insbesondere kein Amin-terminiertes Präpolymer enthaltend Polyalkylenoxideinheiten, enthalten ist. Derartige Amin-terminierte Präpolymere sind im Handel erhältlich (zum Beispiel als Jeffamin® ED-2003; Huntsman, Everberg, Belgien) und werden anstelle von dem Stärkederivat im Stand der Technik häufig mit Isocyanat-terminierten Präpolymeren zur Reaktion gebracht, um Hydrogele zu erhalten. Vorliegend wird aber der Ansatz verfolgt, das Isocyanat-terminierte Präpolymer mit einem Naturstoffderivat, nämlich einem Stärkederivat, und nicht mit einem vollständig künstlich hergestellten Amin-terminierten Präpolymer umzusetzen, um ein biokompatibleres, nachhaltigeres und umweltverträglicheres Polymer erhalten zu können.

Es kann auch vorteilhafterweise vorgesehen sein, dass in der Reaktionsmischung gar keine Komponente mit einer Amin-Gruppe enthalten ist. Die Reaktionsmischung kann dann auch das zuvor genannte Amin-terminierte Präpolymer nicht enthalten. Alternativ oder zusätzlich dazu kann vorgesehen sein, dass in der Reaktionsmischung außer dem Stärkederivat keine Komponente mit einer Hydroxyl-Gruppe enthalten ist. Die Reaktionsmischung kann dann jedoch auch die zuvor genannten Feuchthaltemittel nicht enthalten. Wenn die Reaktionsmischung keine Komponente mit einer Amin-Gruppe und/oder außer dem Stärkederivat keine Komponente mit einer Hydroxyl-Gruppe enthält, kann der kovalente Einbau des Stärkederivats in das Polymer besonders gut sichergestellt werden.

In einer weiteren Ausführungsform der Erfindung wird beim Umsetzen des Präpolymers ein Gas in die Reaktionsmischung eingebracht. Vorzugsweise ist das Gas Kohlendioxid. Ähnlich wie mit dem zuvor genannten Treibmittel kann damit die Porosität und in der Folge die Saugkraft des Polymers erhöht werden, was insbesondere bei einem Polymer in der Form eines Schaumstoffs von Interesse sein kann. Außerdem kann die Saugkraft des Polymers dadurch verbessert werden, indem eine oberflächliche Schicht des Polymers abgetragen wird, so dass eine poröse Struktur des Polymers freigelegt wird. Das Herstellungsverfahren umfasst dann einen zusätzlichen Schritt, welcher sich an den erfindungsgemäßen Schritt iv. anschließt und in dem eine oberflächliche Schicht des Polymers abgetragen wird. Dieser zusätzliche Verfahrensschritt kommt gleichfalls wieder vor allem dann infrage, wenn das Polymer als Schaumstoff vorliegt.

Das Polymer wurde vorliegend für die Medizin und insbesondere für die Wundbehandlung entwickelt. Entsprechend wird vorliegend mit Anspruch 12 auch die Verwendung des Polymers in allen seinen Ausführungsformen in der Medizin (1. medizinische Indikation) sowie insbesondere in der Wundbehandlung (2. medizinische Indikation) beansprucht.

Bei der Verwendung des Polymers in der Wundbehandlung wird dieses meistens in einen Wundverband integriert. Entsprechend wird vorliegend mit Anspruch 13 auch ein Wundverband mit dem Polymer in allen seinen Ausführungsformen beansprucht. Ein solcher Wundverband kann zum Beispiel eine Rückschicht und eine wundkontaktierende Schicht umfassen, wobei das Polymer die wundkontaktierende Schicht bildet. Dann kann das Polymer seine vorteilhaften Eigenschaften wie zum Beispiel die Absorption von Wundexsudat am besten ausüben.

## Beispiele und Figuren

Mit den nachfolgend beschriebenen Beispielen und Figuren soll die Erfindung exemplarisch näher erläutert werden.

### Herstellung der Polymere

Es wurden verschiedene Polymere hergestellt und deren Eigenschaften untersucht. **Tabelle 1** listet die Zusammensetzung und Konsistenz der hergestellten Polymere bestehend aus Präpolymer, Stärkederivat und Wasser auf.

Tabelle 1: Zusammensetzung und Konsistenz der hergestellten Polymere

| Beispiel Nummer | Stocklösung [g] | Präpolymer [Gew.-%] | Stärkederivat [Gew.-%] | Wasser [Gew.-%] | Konsistenz |
|---|---|---|---|---|---|
| 1 | 1,5 | 76,92 | 0,62 | 22,46 | Schaumstoff |
| 2 | 2,5 | 66,67 | 0,89 | 32,44 | Schaumstoff |
| 3 | 5,0 | 50,00 | 1,33 | 48,67 | Schaumstoff |
| 4 | 7,5 | 40,00 | 1,60 | 58,40 | Gelschaum |
| 5 | 10,0 | 33,33 | 1,78 | 64,89 | Gel |
| 6 | 12,5 | 28,57 | 1,90 | 69,52 | Gel |
| 7 | 15,0 | 25,00 | 2,00 | 73,00 | Gel |
| 8 | 4,96 | 50,20 | 1,33 | 48,47 | Schaumstoff |
| 9 | 8,09 | 38,20 | 1,65 | 60,15 | Gelschaum |
| 10 | 11,16 | 30,94 | 1,84 | 67,22 | Gel |
| 11 | 14,12 | 26,15 | 1,97 | 71,88 | Gel |
| 12 | 17,18 | 22,54 | 2,07 | 75,39 | Gel |
| 13 | 20,20 | 19,84 | 2,14 | 78,02 | Gel |

Die Herstellung der Polymere soll anhand von Beispiel 3 näher erläutert werden:

Schritt 1 (Herstellung der anspruchsgemäßen wässrigen, stärkederivathaltigen Zubereitung): 2 g Stärkederivat wurden in 75 ml demineralisiertem Wasser (anspruchsgemäße wässrige Flüssigkeit) gelöst. Entsprechend besaß die Zubereitung eine Stärkederivat-Konzentration von 2,67 Gew.-%.
Schritt 2 (Herstellung der anspruchsgemäßen Reaktionsmischung):
5 g Präpolymer wurden mit 5 g der in Schritt 1 hergestellten Zubereitung (in Tabelle 1 als "Stocklösung" bezeichnet) gemischt. Die dabei erhaltene Reaktionsmischung bestand demnach aus 5,0 g Präpolymer, 0,13 g Stärkederivat und 4,87 g Wasser. Die Anteile an dem Präpolymer, dem Stärkederivat und dem Wasser bezogen auf das Gesamtgewicht der Reaktionsmischung betrugen folglich 50,0 Gew.-%, 1,33 Gew.-% beziehungsweise 48,67 Gew.-%. Die Reaktionsmischung wurde in eine Petrischale eingebracht. Es wurden auch größere Mengen der Reaktionsmischung hergestellt, wenn mehrere Petrischalen befüllt werden sollten oder eine Petrischale vollständig mit der Reaktionsmischung zu befüllen war.
Schritt 3 (Erhalt des Polymers):

Die Reaktionsmischung wurde in der Petrischale ruhen gelassen, bis der Polymerisationsprozess und somit die Umsetzung des Präpolymers abgeschlossen war. Der Polymerisationsprozess wurde als abgeschlossen angesehen, wenn die Probe verfestigt war (also den Gelpunkt erreicht hatte) und an ihrer Oberfläche nicht mehr klebrig war. Dies konnte im Labormaßstab mit einem Spatel manuell überprüft werden.

Die Beispiele 1, 2 und 4 bis 7 in Tabelle 1 wurden in ähnlicher Weise wie Beispiel 3 hergestellt. Der Unterschied bei der Herstellung dieser Beispiele beschränkte sich auf die Menge an Stocklösung, die in Schritt 2 mit dem Präpolymer gemischt wurde. So wurden bei Beispiel 5 10 g Stocklösung mit 5 g Präpolymer gemischt, um die Reaktionsmischung zu erhalten.

Die Beispiele 8 bis 13 in Tabelle 1 wurden gleichfalls ähnlich wie Beispiel 3 hergestellt. Neben anderen Mengen an Stocklösung unterschieden sich diese Beispiele von Beispiel 3 zusätzlich dadurch, dass die Stocklösung und das Präpolymer nicht von Hand, sondern mittels einer Laborgelgießanlage gemischt und ausgegossen wurden. Dabei kam die Laborgelgießanlage B100 von der Firma bdtronic zum Einsatz. Das heißt, die Beispiele 1 bis 7 wurden vollständig manuell hergestellt, wohingegen für die Herstellung der Beispiele 8 bis 13 die zuvor genannte Laborgelgießanlage zum Einsatz kam, um Präpolymer und Stocklösung zu mischen und die dabei gebildete Mischung in die Petrischale zu gießen.

Die folgenden vier Angaben treffen auf alle Beispiele aus Tabelle 1 zu. Auf die Beispiele aus Tabelle 2 treffen nur die ersten drei Angaben zu.

- Die Flüssigkeit zum Auflösen des Stärkederivats bestand aus demineralisiertem Wasser.
- Das Stärkederivat war Explotab PCF von JRS Pharma (Rosenberg, Deutschland). Dabei handelt es sich um ein Natriumstärkeglycolat.
- Das Präpolymer war Aquapol® PI-13000-31 von Carpenter (Richmond, USA). Wie bereits erwähnt, handelt es sich dabei um ein dreiarmig verzweigtes Isocyanat-terminiertes Präpolymer enthaltend Polyethylenoxid- und Polypropylenoxideinheiten.
- Die Reaktionsmischung enthielt nur das Präpolymer und die wässrige, stärkederivathaltige Zubereitung.

Die Polymere aus **Tabelle 2** enthalten neben Präpolymer, Stärkederivat und Wasser noch eine oder mehrere weitere Komponenten wie ein Feuchthaltemittel und/oder ein anorganisches Salz.

Tabelle 2: Zusammensetzung von Polymeren mit Feuchthaltemittel und/oder Salz

| Beispiel Nummer | Präpolymer Gew.-% | Stärkederivat Gew.-% | Wasser Gew.-% | Feuchthaltemittel Gew.-% | Salz Gew.-% |
|---|---|---|---|---|---|
| 14 | 41,67 | 1,08 | 40,58 | 16,67 | - |
| 15 | 49,02 | 1,27 | 47,75 | - | 1,96 |
| 16 | 40,98 | 1,07 | 39,92 | 16,39 | 1,64 |

Die Beispiele 14 bis 16 sind ähnlich zusammengesetzt wie das Beispiel 3. Das Beispiel 14 unterscheidet sich von Beispiel 3 dadurch, dass es zusätzlich ein Feuchthaltemittel enthält. Das Beispiel 15 enthält im Unterschied zu Beispiel 3 ein anorganisches Salz. Das Beispiel 16 enthält im Unterschied zu Beispiel 3 sowohl ein Feuchthaltemittel als auch ein anorganisches Salz. Als Feuchthaltemittel können zum Beispiel Ethylenglykol, Propylenglykol, PEG300, PEG2000, Glycerol, Saccharose oder Sorbitol verwendet werden. Als anorganisches Salz kann insbesondere Natriumchlorid verwendet werden.

Die Beispiele 14 bis 16 konnten ganz ähnlich wie Beispiel 3 hergestellt werden. Die wässrige, stärkederivathaltige Zubereitung wurde wie zuvor in Schritt 1 beschrieben hergestellt. Anschließend wurden 5 g Präpolymer, 5 g der stärkederivathaltigen Zubereitung und die weiteren Komponenten (2 g Feuchthaltemittel bei den Beispielen 14 und 16 und 0,2 g Salz bei den Beispielen 15 und 16) miteinander gemischt, in eine Petrischale gegossen und aushärten gelassen. Die verwendeten Feuchthaltemittel sowie das eingesetzte Salz ließen sich aufgrund ihrer hohen Wasserlöslichkeit und insbesondere bei Ansatz größerer Mengen der Reaktionsmischung gut mit dem Präpolymer und der stärkederivathaltigen Zubereitung mischen. Ein größerer Ansatz der Reaktionsmischung konnte bezogen auf Polymer 16 beispielsweise durch Mischen von 25 g Präpolymer, 25 g stärkederivathaltige Zubereitung, 10 g Feuchthaltemittel, zum Beispiel 10 g Glycerol, und 1 g Salz, zum Beispiel 1 g Natriumchlorid, erhalten werden (= 5-facher Ansatz).

Konsistenz der Polymere

Die **Figuren 1 und 2** zeigen Fotografien der Beispiele 1 bis 7 beziehungsweise 8 bis 13 aus Tabelle 1. Die Figuren sollen veranschaulichen, wie das Polymer als Gel, Gelschaum und Schaumstoff aussehen kann. Die Nummern der

Beispiele sind unter den jeweiligen Proben gekennzeichnet. In Figur 1 beziehen sich die auf den oberen Rand der Petrischalen geschriebenen Zahlenwerte ersichtlich auf Spalte 2 von Tabelle 1. In Figur 2 haben die auf den oberen Rand der Petrischalen geschriebenen prozentualen Angaben jedoch keinen direkten Bezug zu den Werten in Tabelle 1, sondern beziehen sich auf Pumpendrehzahlen. So wurden in Beispiel 8 die Dosierpumpen der Gelgießanlage für 14 Sekunden aktiviert, wobei die Dosierpumpe für das Präpolymer mit 95 % ihrer maximalen Drehzahl und die Dosierpumpe für die Stocklösung mit 10 % ihrer maximalen Drehzahl angesteuert wurde. Dies entsprach dann einer Menge an Präpolymer von 5 g und einer Menge an Stocklösung von 4,96 g, die von der Gelgießanlage miteinander gemischt und in die Petrischale zum Aushärten gegossen wurden. Bei den Beispielen 9 bis 13 wurde im Vergleich zu Beispiel 8 lediglich die Drehzahl der Dosierpumpe für die Stocklösung erhöht, um die Menge der mit dem Präpolymer gemischten Stocklösung zu erhöhen. Die eingestellte Pumpendrehzahl als indirektes Maß der verwendeten Menge an Stocklösung wurde wie in Figur 2 zu sehen ist zur Unterscheidung der Proben auf die Deckel der Petrischalen geschrieben.

Die Beispiele 1 bis 3 bildeten vergleichsweise harte, weiße Schaumstoffe. Beispiel 4 bildete einen weißen Gelschaum. Die Beispiele 5 bis 7 bildeten schließlich zunehmend durchsichtig und weicher werdende Gele. Die Polymere wurden also von Beispiel 1 zu Beispiel 7 zunehmend durchsichtiger und weicher. Beispiel 8 ergab einen weißen Schaumstoff und Beispiel 9 einen weißen Gelschaum, vergleichbar mit den Beispielen 3 beziehungsweise 4. Die Beispiele 10 bis 13 ergaben ähnlich wie die Beispiele 5 bis 7 Gele. Die Bilder in den Figuren 1 und 2 veranschaulichen zudem, dass die Homogenität der Polymere durch die Verwendung der Laborgelgießanlage deutlich verbessert werden konnte.

Die Herstellung der Beispiele 1 bis 13 hat gezeigt, dass letztlich das Mengenverhältnis Präpolymer zu wässriger Flüssigkeit darüber entscheidet, ob ein Polymer in Form eines Gels, eines Gelschaums oder eines Schaumstoffs erhalten wird. So geht das Polymer von einem gelartigen zu einem schaumstoffartigen Zustand über, wenn die Präpolymerkonzentration in der Reaktionsmischung erhöht und die Wassermenge in der Reaktionsmischung erniedrigt wird. Entsprechend geht das Polymer von einem schaumstoffartigen zu einem gelartigen Zustand über, wenn die Präpolymerkonzentration in der Reaktionsmischung erniedrigt und die Wassermenge in der Reaktionsmischung erhöht wird.

Absorptionskapazität der Polymere

Die Beispiele 1 bis 7 aus Tabelle 1 wurden hinsichtlich ihrer Absorptionskapazität untersucht. Zur Messung der Absorptionskapazität wurden Probenstücke mit einer Größe von 3 cm x 3 cm ausgestanzt und gewogen. Anschließend wurden sie in ein Becherglas mit demineralisiertem Wasser für 30 Minuten, 60 Minuten, 90 Minuten und 120 Minuten gegeben. Daraufhin wurden sie erneut gewogen. Die Berechnung der Absorptionskapazität erfolgt nach folgender Gleichung und wird in der Einheit g/g angegeben:

$$\text{Absorptionskapazität} = (\text{Endgewicht} - \text{Anfangsgewicht}) / \text{Anfangsgewicht}$$

**Figur 3** zeigt die Ergebnisse der Absorptionstests für die Beispiele 1 bis 7 aus Tabelle 1. Alle untersuchten Proben haben für die Anwendung in der Wundtherapie zufriedenstellende Absorptionskapazitäten gezeigt. Wenn allerdings ein möglichst hohes Absorptionsvermögen erwünscht ist (zum Beispiel wenn stark exsudierende Wunden behandelt werden sollen), können die Schaumstoff-Polymere (Beispiele 1 bis 3 und 8) vorgezogen werden.

IR-Spektren der Polymere und der Ausgangsstoffe

**Figur 4** zeigt FT-IR-Spektren von dem eingesetzten Stärkederivat (Explotab PCF) und Präpolymer (Aquapol®) sowie von ausgewählten Beispielen aus Tabelle 1. In der Figur sind die Spektren folgender Proben zu sehen (von oben nach unten): Stärkederivat, Präpolymer, Beispiel 1, Beispiel 4, Beispiel 7. Die hergestellten Polymere (Beispiele 1, 4 und 7) wurden für die Aufnahme der FT-IR-Spektren getrocknet.

Isocyanat-Gruppen, Urethan-Gruppen und Harnstoff-Gruppen führen in den IR-Spektren zu Absorptionsbanden bei 2260 $cm^{-1}$, 1714 $cm^{-1}$ beziehungsweise 1654 $cm^{-1}$. Die aufgenommenen IR-Spektren belegen, dass die hergestellten Polymere keine Isocyanat-Gruppen mehr besitzen und somit eine vollständige Umsetzung der Präpolymere stattfand.

Wundverband mit dem Polymer

**Figur 5** zeigt einen Wundverband mit einem erfindungsgemäßen Polymer in schematischer Form. Der Wundverband umfasst eine Rückschicht **1,** welche mit einer Klebeschicht **2** vollflächig ausgerüstet ist. Die Rückschicht **1** kann zum Beispiel ein wasserundurchlässiger, wasserdampfdurchlässiger Polyurethanfilm sein, welcher vollflächig mit einem Acrylatkleber als Klebeschicht **2** beschichtet ist. Die Rückschicht **1** dient als tragende und abdeckende Lage in dem Wundverband. Die Klebeschicht **2** ermöglicht die Befestigung des Wundverbands am Körper des Patienten. Weiterhin

umfasst der Wundverband eine Wundkontaktschicht **3,** welche von dem erfindungsgemäßen Polymer gebildet wird. Beim Befestigen des Wundverbandes am Körper des Patienten wird die Wundkontaktschicht **3** direkt auf der Wunde platziert. Mit der Wundkontaktschicht **3** kann der Wundverband Wundexsudat absorbieren und Feuchtigkeit an die Wunde abgeben.

Es ist auch denkbar, dass der Wundverband noch weitere Schichten umfasst. Zum Beispiel kann eine weitere, absorbierende Schicht zwischen der Klebeschicht **2** und der Wundkontaktschicht **3** angeordnet werden, um die Absorptionskapazität des Wundverbandes zu steigern. Die absorbierende Schicht kann dabei beispielsweise einen hydrophilen Polyurethanschaumstoff oder einen Vliesstoff umfassen.

Der Wundverband kann hergestellt werden, indem das Polymer in flüssigem Zustand (also die anspruchsgemäße Reaktionsmischung) auf die Rückschicht **1** beziehungsweise deren Klebeschicht **2** direkt aufgegossen wird. Der fertige Wundverband liegt dann nach dem Aushärten des Polymers vor. Alternativ kann die Wundkontaktschicht **3** auch in einer separaten Gießform hergestellt und dann mit der Rückschicht **1** über deren Klebeschicht **2** verbunden werden.

## Patentansprüche

**1.** Verfahren zur Herstellung eines Polymers für medizinische Zwecke, insbesondere für die Wundbehandlung, umfassend die Schritte

i. Bereitstellen eines Isocyanat-terminierten Präpolymers enthaltend Polyalkylenoxideinheiten,
ii. Lösen eines Stärkederivats in einer wässrigen Flüssigkeit, um eine wässrige, stärkederivathaltige Zubereitung zu erhalten,
iii. Mischen des Präpolymers, der wässrigen, stärkederivathaltigen Zubereitung und optional einer oder mehrerer weiterer Komponenten, um eine Reaktionsmischung zu erhalten,
iv. Umsetzen des Präpolymers in der Reaktionsmischung, um das Polymer zu erhalten,

wobei

- der Anteil an dem Präpolymer bezogen auf das Gesamtgewicht der Reaktionsmischung 5 bis 80 Gew.-%, insbesondere 20 bis 80 Gew.-%, beträgt,
- der Anteil an dem Stärkederivat bezogen auf das Gesamtgewicht der Reaktionsmischung 0,5 bis 5 Gew.-%, insbesondere 0,5 bis 2 Gew.-%, beträgt,
- der Anteil der wässrigen Flüssigkeit bezogen auf das Gesamtgewicht der Reaktionsmischung 20 bis 90 Gew.-%, insbesondere 20 bis 75 Gew.-%, beträgt, und
- der Anteil der einen oder mehreren weiteren Komponenten bezogen auf das Gesamtgewicht der Reaktionsmischung 0 bis 30 Gew.-% beträgt,

und wobei das Polymer als ein Gel, ein Gelschaum oder ein Schaumstoff vorliegt.

**2.** Verfahren nach Anspruch 1, wobei das Präpolymer mindestens dreiarmig verzweigt ist und die Polyalkylenoxideinheiten durch Polyethylenoxid- und/oder Polypropylenoxideinheiten gebildet werden, wobei das Gewichtsverhältnis von Ethylenoxid- zu Propylenoxideinheiten vorzugsweise 3 : 1 bis 7 : 1 beträgt.

**3.** Verfahren nach Anspruch 1 oder 2, wobei es sich bei dem Stärkederivat um Natriumstärkeglycolat handelt.

**4.** Verfahren nach einem der vorangehenden Ansprüche, wobei ein Feuchthaltemittel als weitere Komponente vorhanden ist, wobei das Feuchthaltemittel vorzugsweise Ethylenglykol, Propylenglykol, PEG300, PEG2000, Glycerol, Saccharose oder Sorbitol ist.

**5.** Verfahren nach einem der vorangehenden Ansprüche, wobei ein anorganisches Salz als weitere Komponente vorhanden ist, wobei das Salz vorzugsweise Natriumchlorid, Kaliumchlorid, Magnesiumchlorid, Calciumchlorid oder eine Mischung aus mindestens zwei dieser Salze ist.

**6.** Verfahren nach einem der Ansprüche 1 bis 5, wobei

- der Anteil an dem Präpolymer bezogen auf das Gesamtgewicht der Reaktionsmischung 5 bis 35 Gew.-% beträgt, und
- der Anteil der wässrigen Flüssigkeit bezogen auf das Gesamtgewicht der Reaktionsmischung 65 bis 90 Gew.-%

beträgt.

**7.** Verfahren nach einem der Ansprüche 1 bis 5, wobei

- der Anteil an dem Präpolymer bezogen auf das Gesamtgewicht der Reaktionsmischung 36 bis 45 Gew.-% beträgt, und
- der Anteil der wässrigen Flüssigkeit bezogen auf das Gesamtgewicht der Reaktionsmischung 55 bis 60 Gew.-% beträgt.

**8.** Verfahren nach einem der Ansprüche 1 bis 5, wobei

- der Anteil an dem Präpolymer bezogen auf das Gesamtgewicht der Reaktionsmischung 50 bis 80 Gew.-% beträgt, und
- der Anteil der wässrigen Flüssigkeit bezogen auf das Gesamtgewicht der Reaktionsmischung 22 bis 50 Gew.-% beträgt.

**9.** Verfahren nach einem der vorangehenden Ansprüche, wobei in der Reaktionsmischung kein Amin-terminiertes Präpolymer, insbesondere kein Amin-terminiertes Präpolymer enthaltend Polyalkylenoxideinheiten, enthalten ist.

**10.** Verfahren nach einem der vorangehenden Ansprüche, wobei das Verfahren einen zusätzlichen Schritt umfasst, wobei in dem zusätzlichen Schritt eine oberflächliche Schicht des Polymers abgetragen wird.

**11.** Polymer, erhältlich durch ein Verfahren nach einem der vorangehenden Ansprüche.

**12.** Polymer nach Anspruch 11 zur Anwendung in der Medizin, insbesondere zur Anwendung in der Wundbehandlung.

**13.** Wundverband, umfassend ein Polymer nach Anspruch 11.

**14.** Wundverband nach Anspruch 13, wobei der Wundverband eine Rückschicht (1) und eine wundkontaktierende Schicht (3) umfasst, wobei das Polymer die wundkontaktierende Schicht (3) bildet.

**Claims**

**1.** Method for producing a polymer for medical purposes, in particular for wound treatment, comprising the steps of

i. providing an isocyanate-terminated prepolymer containing polyalkylene oxide units,
ii. dissolving a starch derivative in an aqueous liquid to obtain an aqueous preparation containing starch derivative,
iii. mixing the prepolymer, the aqueous preparation containing starch derivative, and optionally one or more further components to obtain a reaction mixture,
iv. reacting the prepolymer in the reaction mixture to obtain the polymer,

wherein

- the proportion of the prepolymer in relation to the total weight of the reaction mixture is 5 to 80% by weight, in particular 20 to 80% by weight,
- the proportion of the starch derivative in relation to the total weight of the reaction mixture is 0.5 to 5% by weight, in particular 0.5 to 2% by weight,
- the proportion of the aqueous liquid in relation to the total weight of the reaction mixture is 20 to 90% by weight, in particular 20 to 75% by weight, and
- the proportion of the one or more further components in relation to the total weight of the reaction mixture is 0 to 30% by weight,

and wherein the polymer is present as a gel, a gel foam or a foam.

**2.** Method according to Claim 1, wherein the prepolymer has at least three-armed branching and the polyalkylene oxide units are formed by polyethylene oxide and/or polypropylene oxide units, wherein the weight ratio of ethylene oxide to

propylene oxide units is preferably 3: 1 to 7: 1.

**3.** Method according to Claim 1 or 2, wherein the starch derivative is sodium starch glycolate.

**4.** Method according to any of the preceding claims, wherein a humectant is present as a further component, wherein the humectant is preferably ethylene glycol, propylene glycol, PEG300, PEG2000, glycerol, sucrose or sorbitol.

**5.** Method according to any of the preceding claims, wherein an inorganic salt is present as a further component, wherein the salt is preferably sodium chloride, potassium chloride, magnesium chloride, calcium chloride or a mixture of at least two of these salts.

**6.** Method according to any of Claims 1 to 5, wherein

- the proportion of the prepolymer in relation to the total weight of the reaction mixture is 5 to 35% by weight, and
- the proportion of the aqueous liquid in relation to the total weight of the reaction mixture is 65 to 90% by weight.

**7.** Method according to any of Claims 1 to 5, wherein

- the proportion of the prepolymer in relation to the total weight of the reaction mixture is 36 to 45% by weight, and
- the proportion of the aqueous liquid in relation to the total weight of the reaction mixture is 55 to 60% by weight.

**8.** Method according to any of Claims 1 to 5, wherein

- the proportion of the prepolymer in relation to the total weight of the reaction mixture is 50 to 80% by weight, and
- the proportion of the aqueous liquid in relation to the total weight of the reaction mixture is 22 to 50% by weight.

**9.** Method according to any of the preceding claims, wherein no amine-terminated prepolymer, in particular no amine-terminated prepolymer containing polyalkylene oxide units, is contained in the reaction mixture.

**10.** Method according to any of the preceding claims, wherein the method comprises an additional step, wherein in the additional step a superficial layer of the polymer is removed.

**11.** Polymer obtainable by a method according to any of the preceding claims.

**12.** Polymer according to Claim 11 for use in medicine, in particular for use in wound treatment.

**13.** Wound dressing comprising a polymer according to Claim 11.

**14.** Wound dressing according to Claim 13, wherein the wound dressing comprises a backing layer (1) and a wound-contacting layer (3), wherein the polymer forms the wound-contacting layer (3).

**Revendications**

**1.** Procédé de préparation d'un polymère destiné à des fins médicales, en particulier pour le traitement de plaies, comprenant les étapes

i. mise à disposition d'un prépolymère terminé par isocyanate contenant des motifs poly(oxyde d'alkylène),
ii. dissolution d'un dérivé d'amidon dans un liquide aqueux afin d'obtenir une préparation aqueuse, contenant un dérivé d'amidon,
iii. mélange du prépolymère, de la préparation aqueuse contenant un dérivé d'amidon et éventuellement d'un ou de plusieurs composants afin d'obtenir un mélange réactionnel,
iv. transformation du prépolymère dans le mélange réactionnel, afin d'obtenir le polymère,

dans lequel

- la proportion de prépolymère par rapport au poids total du mélange réactionnel représente 5 à 80% en poids, en particulier 20 à 80% en poids,

- la proportion de dérivé d'amidon par rapport au poids total du mélange réactionnel représente 0,5 à 5% en poids, en particulier 0,5 à 2% en poids,
- la proportion de liquide aqueux par rapport au poids total du mélange réactionnel représente 20 à 90% en poids, en particulier 20 à 75% en poids et
- la proportion dudit un ou desdits plusieurs autres composants par rapport au poids total du mélange réactionnel représente 0 à 30% en poids,

et dans lequel le polymère se trouve sous forme d'un gel, d'une mousse de gel ou d'une mousse.

**2.** Procédé selon la revendication 1, dans lequel le prépolymère est ramifié en au moins trois bras et les motifs de poly(oxyde d'alkylène) sont formés par des motifs de poly(oxyde d'éthylène) et/ou des motifs de poly(oxyde de propylène), le rapport en poids des motifs d'oxyde d'éthylène aux motifs d'oxyde de propylène représentant de préférence 3:1 à 7:1.

**3.** Procédé selon la revendication 1 ou 2, dans lequel il s'agit, pour le dérivé d'amidon, de glycolate d'amidon sodique.

**4.** Procédé selon l'une des revendications précédentes, dans lequel un agent de rétention d'humidité est présent comme autre composant, l'agent de rétention d'humidité étant de préférence l'éthylèneglycol, le propylèneglycol, le PEG300, le PEG2000, le glycérol, le saccharose ou le sorbitol.

**5.** Procédé selon l'une des revendications précédentes, dans lequel un sel inorganique est présent comme autre sel, le sel étant de préférence le chlorure de sodium, le chlorure de potassium, le chlorure de magnésium, le chlorure de calcium ou un mélange d'au moins deux de ces sels.

**6.** Procédé selon l'une des revendications 1 à 5, dans lequel

- la proportion de prépolymère par rapport au poids total du mélange réactionnel représente 5 à 35% en poids et
- la proportion de liquide aqueux par rapport au poids total du mélange réactionnel représente 65 à 90% en poids.

**7.** Procédé selon l'une des revendications 1 à 5, dans lequel

- la proportion de prépolymère par rapport au poids total du mélange réactionnel représente 36 à 45% en poids et
- la proportion de liquide aqueux par rapport au poids total du mélange réactionnel représente 55 à 60% en poids.

**8.** Procédé selon l'une des revendications 1 à 5, dans lequel

- la proportion de prépolymère par rapport au poids total du mélange réactionnel représente 50 à 80% en poids et
- la proportion de liquide aqueux par rapport au poids total du mélange réactionnel représente 22 à 50% en poids.

**9.** Procédé selon l'une des revendications précédentes, dans lequel le mélange réactionnel ne contient pas de prépolymère terminé par amine, en particulier pas de prépolymère terminé par amine contenant des motifs poly(oxyde d'alkylène).

**10.** Procédé selon l'une des revendications précédentes, dans lequel le procédé comprend une étape supplémentaire, une couche superficielle du polymère étant éliminée dans l'étape supplémentaire.

**11.** Polymère, pouvant être obtenu par un procédé selon l'une des revendications précédentes.

**12.** Polymère selon la revendication 11 destiné à être utilisé en médecine, en particulier dans le traitement de plaies.

**13.** Pansement, comprenant un polymère selon la revendication 11.

**14.** Pansement selon la revendication 13, le pansement comprenant une couche arrière (1) et une couche (3) en contact avec la plaie, le polymère formant la couche (3) en contact avec la plaie.

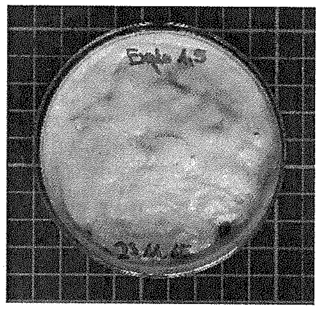

Beispiel 1

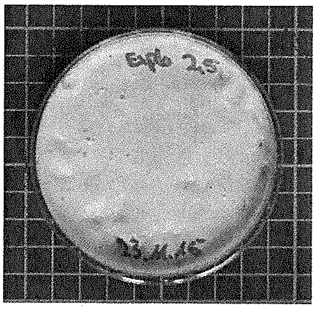

Beispiel 2

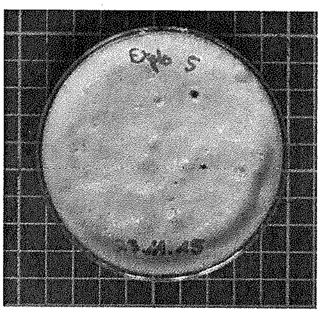

Beispiel 3

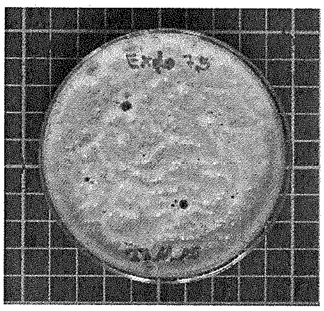

Beispiel 4

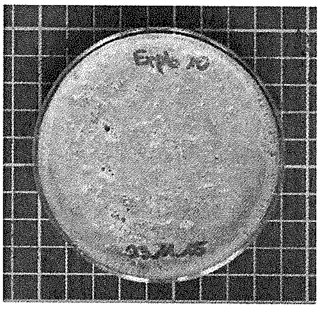

Beispiel 5

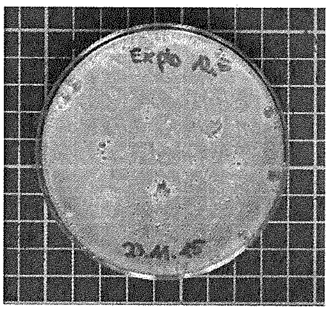

Beispiel 6

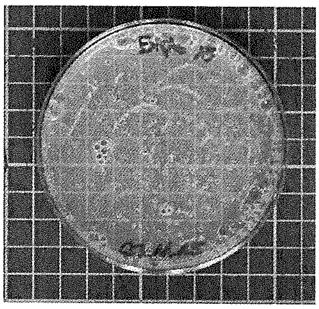

Beispiel 7

Figur 1

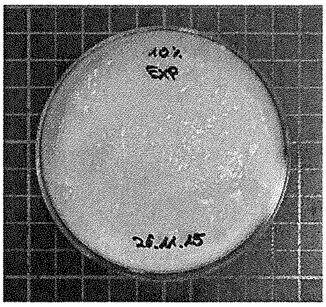

Beispiel 8

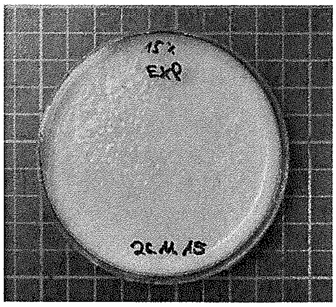

Beispiel 9

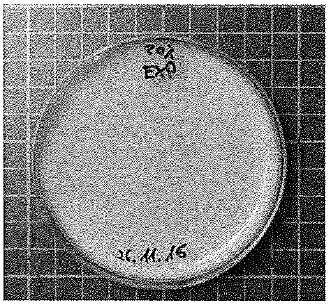

Beispiel 10

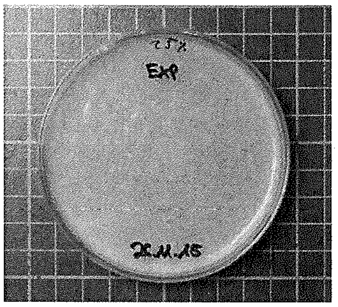

Beispiel 11

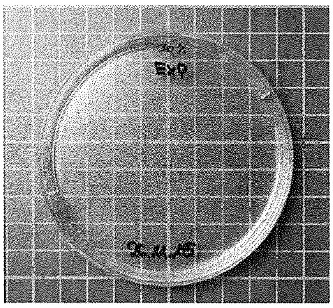

Beispiel 12

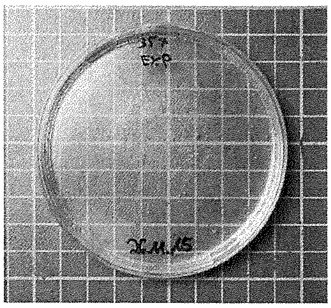

Beispiel 13

Figur 2

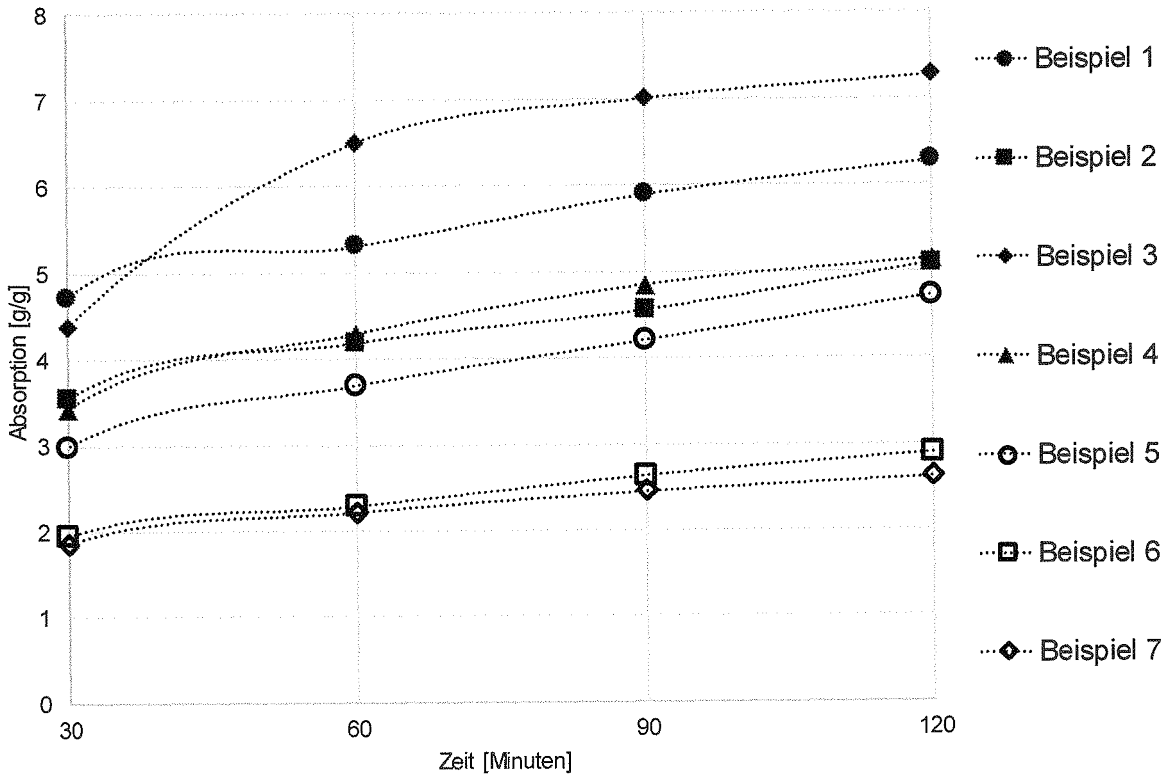
8
7
6
5
4
3
2
1
0
Absorption [g/g]
30
60
90
120
Zeit [Minuten]
Beispiel 1
Beispiel 2
Beispiel 3
Beispiel 4
Beispiel 5
Beispiel 6
Beispiel 7

Figur 3

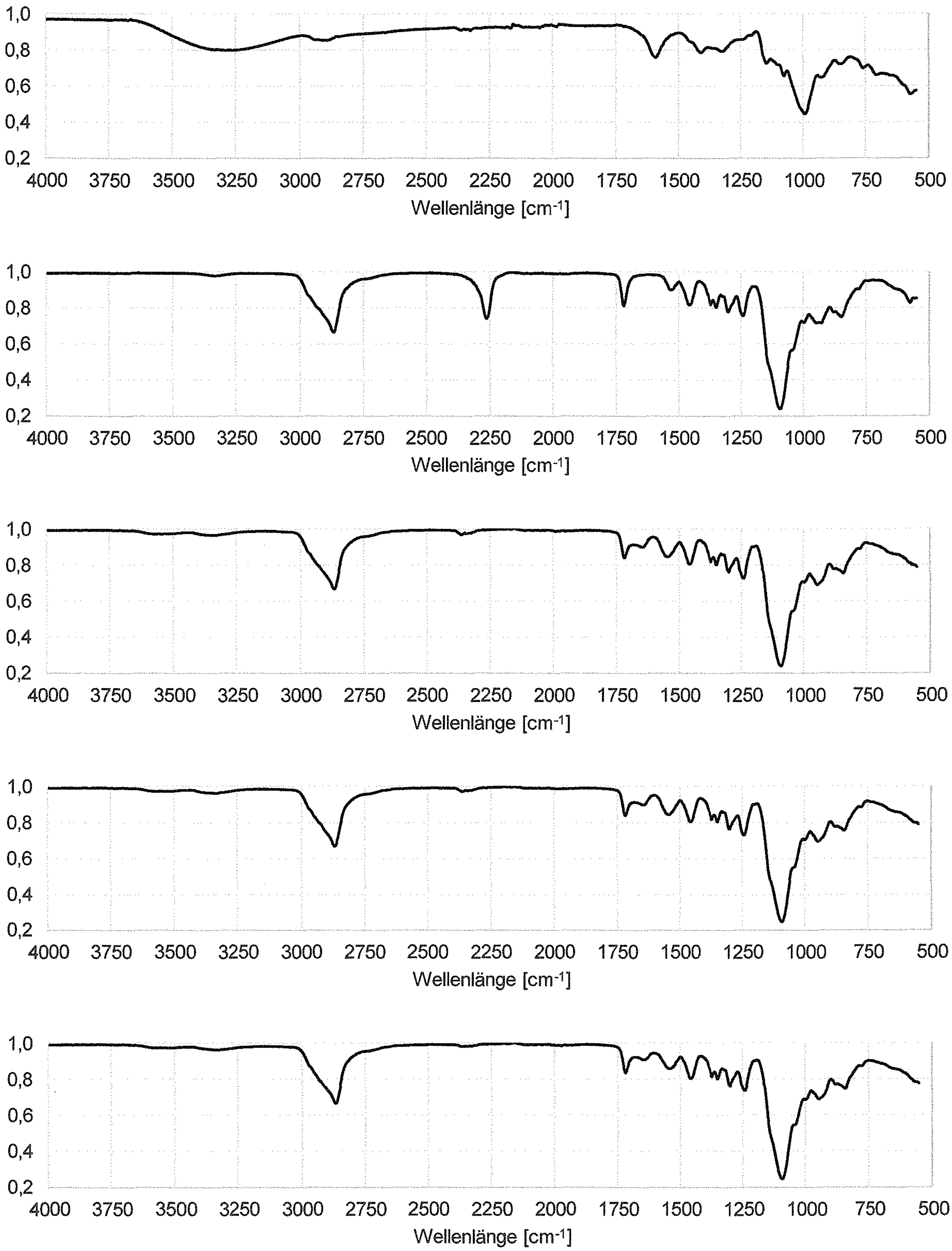
1,0
0,8
0,6
0,4
0,2
4000 3750 3500 3250 3000 2750 2500 2250 2000 1750 1500 1250 1000 750 500
Wellenlänge [cm-1]
Wellenlänge [cm-1]
Wellenlänge [cm-1]
Wellenlänge [cm-1]
Wellenlänge [cm-1]

Figur 4

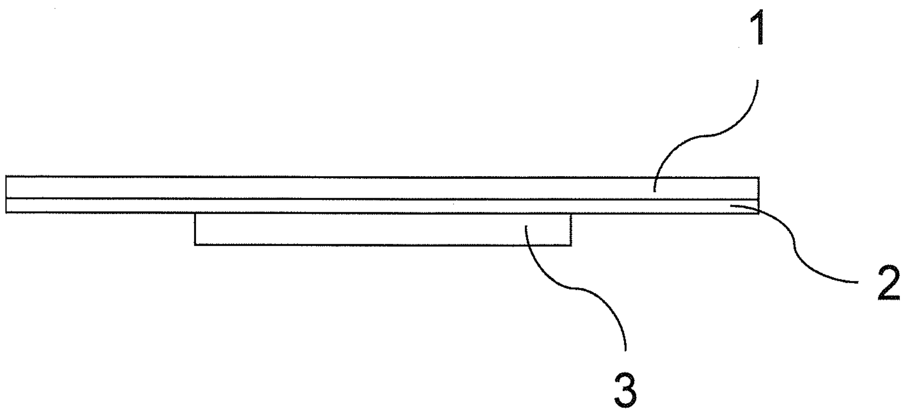


Figur 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- CN 110577627 A
- KR 101879643 B1
- EP 3235520 A1
- JP 2010254985 A
- US 7538257 B2
- US 5065752 A
- US 4773409 A

### In der Beschreibung aufgeführte Nicht-Patentliteratur

- *CHEMICAL ABSTRACTS*, 9063-38-1